# EUROPEAN PATENT APPLICATION

(11) **EP 4 678 751 A1**
(43) Date of publication of application: **14.01.2026**
(21) Application number: 24781298.5
(22) Date of filing: 29.03.2024
(51) Int. Cl.: C12N 15/81, C12N 9/90, C12N 9/10, C12N 9/02, C07K 14/39, C12P 23/00

(54) **MICROORGANISM WITH REINFORCED SNQ2 PROTEIN ACTIVITY AND RETINOID PRODUCING ABILITY AND METHOD FOR PRODUCING RETINOID USING SAME**

(30) Priority: 29.03.2023 KR 20230041432
(71) Applicant: CJ Cheiljedang Corporation, Seoul 04560 (KR)
(72) Inventor: PARK, Hye Min, Seoul 04560 (KR); LEE, Peter, Seoul 04560 (KR); KIM, Jae Eung, Seoul 04560 (KR); LEE, Dong Pil, Seoul 04560 (KR)
(74) Representative: Meissner Bolte Partnerschaft mbB
(86) International application number: PCT/KR2024/004039
(87) International publication number: WO 2024/205308

(57) **Abstract**

The present disclosure relates to a *Yarrowia lipolytica* microorganism having retinoid-producing ability with enhanced activity of an SNQ2 protein; a method for producing retinoid using the same; a composition for producing retinoid; a use for retinoid production; and a method for preparing the microorganism.

## Description

### [Technical Field]

The present disclosure relates to a *Yarrowia lipolytica* microorganism having retinoid-producing ability with enhanced activity of an SNQ2 protein; a method for producing retinoid using the same; a composition for producing retinoid; a use for retinoid production; and a method for preparing the microorganism.

### [Background Art]

Retinol, a fat-soluble vitamin, is an essential vitamin involved in eye health for improving night blindness, strengthening immunity, skin health, and the like. Currently, it is produced and marketed mainly by leading global companies through chemical synthesis, but research is being conducted to produce retinol based on microbial fermentation.

To this end, although numerous technologies have been developed for stabilizing the retinol compound itself in a composition or product containing retinol (*e.g.,* U.S. Patent No. 6,858,217), methods for stably increasing retinol production have been only minimally developed.

### [Disclosure]

### [Technical Problem]

A problem to be solved by the present disclosure is to provide a *Yarrowia lipolytica* microorganism having retinoid-producing ability with enhanced activity of an SNQ2 protein; a method for producing retinoid using the same; a composition for producing retinoid; a use for retinoid production; and a method for preparing the microorganism.

### [Technical Solution]

The present disclosure provides a *Yarrowia lipolytica* microorganism having retinoid-producing ability with enhanced activity of an SNQ2 protein.

The present disclosure provides a method for producing retinoid, comprising culturing the microorganism in a medium.

The present disclosure provides a method for preparing the microorganism.

The present disclosure provides a method for increasing retinoid excretion in the microorganism.

The present disclosure provides a composition for producing retinoid, comprising one or more of the microorganism and a culture thereof.

The present disclosure provides a use of the microorganism and/or a culture thereof for retinoid production.

### [Advantageous Effects]

Retinoid can be produced using the microorganism of the present disclosure.

### [Brief Description of the Drawings]

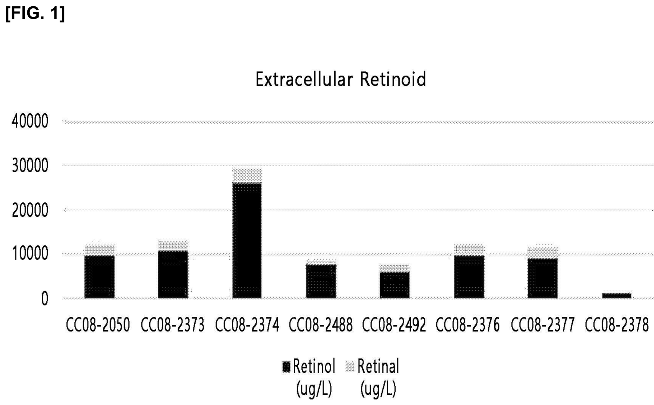

FIG. 1 is a diagram illustrating the retinoid concentration measured in flask culture evaluation of microorganisms.

### [Detailed Description of Preferred Embodiments]

The present disclosure will be described in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below. Furthermore, a number of papers and patent documents are referenced and cited throughout the present specification. The disclosures of the cited papers and patent documents are incorporated herein by reference in their entirety to further clarify the level of art and the description of the present disclosure.

One aspect of the present disclosure provides a *Yarrowia lipolytica* microorganism having retinoid-producing ability with enhanced activity of an SNQ2 protein.

The "SNQ2 protein" of the present disclosure is a type of ATP-binding cassette transporter protein, and the term "ATP-binding cassette transporter" may be used interchangeably with "ABC transporter".

In one embodiment, the SNQ2 protein of the present disclosure may have ABC transporter activity.

In the present disclosure, the "ABC transporter" refers to a protein that belongs to one of the transport system superfamilies, commonly possesses an ATP-binding cassette domain, and utilizes the energy from ATP hydrolysis to perform biological functions (*e.g.,* transporting various substrates across membranes).

The SNQ2 protein of the present disclosure may be capable of enhancing retinoid-producing ability and/or retinoid-excreting ability. In one example, the increase in retinoid-producing ability may be due to an increase in retinoid-excreting ability, but is not limited thereto.

In one embodiment, the activity of the SNQ2 protein of the present disclosure may be enhanced in a *Yarrowia lipolytica* microorganism, thereby increasing the retinoid-producing ability and/or retinoid-excreting ability of the microorganism.

The SNQ2 protein of the present disclosure refers to an endogenous SNQ2 protein of a *Yarrowia lipolytica* microorganism, an SNQ2 protein derived from a *Yarrowia lipolytica* microorganism, or a protein comprising SEQ ID NO: 1 or an amino acid sequence having 70% or more homology thereto.

In one embodiment, the SNQ2 protein of the present disclosure may comprise, have, consist of, or essentially consist of SEQ ID NO: 1 or an amino acid sequence having 90% or more homology or identity thereto.

Specifically, the amino acid sequence of the SNQ2 protein of the present disclosure may be a protein sequence having ABC transporter activity encoded by the SNQ2 gene. The amino acid sequence may be obtained from various databases such as NCBI's GenBank, a publicly known database, but is not limited thereto.

In one embodiment, the SNQ2 protein of the present disclosure may be derived from *Yarrowia lipolytica.*

Further, although an embodiment of the SNQ2 protein of the present disclosure is described as a protein comprising the amino acid sequence of SEQ ID NO: 1, this does not exclude the addition of meaningless sequences upstream or downstream the amino acid sequence of SEQ ID NO: 1, naturally occurring mutations, or silent mutations thereof. Further, it is apparent to those skilled in the art that any protein that exhibits an activity identical or corresponding to that of the protein comprising the amino acid sequence corresponds to the SNQ2 protein of the present disclosure.

Specifically, the SNQ2 protein of the present disclosure may comprise the amino acid sequence of SEQ ID NO: 1, or comprise an amino acid sequence having at least 70%, 75%, 80%, 85%, 86%, 87%, 88%, 89%, 90%, 91%, 92%, 93%, 94%, 95%, 96%, 97%, 98%, or 99% homology or identity thereto. Further, it is apparent that, as long as an amino acid sequence has the above homology or identity and exhibits an efficacy corresponding to that of the protein, it falls within the scope of the present disclosure even if part of the sequence has a deletion, modification, substitution, or addition.

Even if the present disclosure describes a polypeptide (including protein) comprising an amino acid sequence represented by a specific SEQ ID NO, a polypeptide (including protein) consisting of an amino acid sequence represented by a specific SEQ ID NO, or a polypeptide (including protein) having an amino acid sequence represented by a specific SEQ ID NO, it is apparent that a protein having an amino acid sequence with deletion, modification, substitution, or addition in part of the sequence may also be used in the present disclosure, as long as it has an activity identical or corresponding to that of the polypeptide or protein consisting of the amino acid sequence of the corresponding SEQ ID NO. For example, this includes cases of addition of a sequence that does not alter the function of the protein to the N-terminus and/or C-terminus of the amino acid sequence, naturally occurring mutations, silent mutations thereof, or conservative substitutions.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Generally, a conservative substitution may hardly affect or not affect the activity of proteins.

As used herein, the term "homology" or "identity" refers to the degree of similarity between two given amino acid sequences or nucleotide sequences and may be expressed as a percentage. The terms "homology" and "identity" may often be used interchangeably.

The sequence homology or identity of a conserved polynucleotide or polypeptide (including protein) is determined by standard alignment algorithms, and a default gap penalty established by the program used may be used together. Substantially, homologous or identical sequences are generally capable of hybridizing with the whole sequence or a part of the sequence, corresponding to at least about 50%, 60%, 70%, 80%, or 90% of the full length, under moderately or highly stringent conditions. It is apparent that hybridization also comprises hybridization with a polynucleotide containing a general codon or a codon considering codon degeneracy in the polynucleotide.

Whether any two polynucleotide or polypeptide (including protein) sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.] (1988) SIAM J Applied Math 48: 1073)). For example, homology, similarity, or identity may be determined using BLAST of the National Center for Biotechnology Information, or ClustalW.

The homology, similarity, or identity between polynucleotides or polypeptides (including proteins) may be determined by, for example, comparing sequence information using a GAP computer program, such as Needleman et al., (1970), J Mol Biol. 48:443, as described in, for example, Smith and Waterman, Adv. Appl. Math (1981) 2:482. Briefly, a GAP program may be defined as the value acquired by dividing the number of similarly aligned symbols (namely, nucleotides or amino acids) by the total number of symbols in the shorter of two sequences. The default parameters for the GAP program may include: (1) a binary comparison matrix (comprising values of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

Further, whether any two polynucleotide or polypeptide (including protein) sequences have homology, similarity, or identity may be confirmed by comparing sequences through Southern hybridization experiments under stringent conditions, wherein the appropriate hybridization conditions are within the scope of the technical field and can be determined by methods well known to those skilled in the art (such as J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York), but are not limited thereto.

The *Yarrowia lipolytica* microorganism with enhanced activity of an SNQ2 protein of the present disclosure may be a microorganism comprising one or more of: an SNQ2 protein comprising the amino acid sequence of SEQ ID NO: 1 or an amino acid sequence having 70% or more homology thereto; a polynucleotide encoding the same; and a vector comprising the polynucleotide.

As used herein, the term "polynucleotide", which is a polymer of nucleotides in which nucleotide monomers are connected in a long chain shape by covalent bonds, is a DNA strand having a certain length or longer.

The polynucleotide sequence encoding the SNQ2 protein of the present disclosure may be referred to as the "SNQ2 gene", and may comprise a polynucleotide sequence encoding the amino acid sequence represented by SEQ ID NO: 1.

In the polynucleotide, various modifications may be made in the coding region as long as the amino acid sequence of the polypeptide or protein is not changed in consideration of codon degeneracy or codons preferred in organisms that are intended to express the polypeptide or protein. Specifically, the polynucleotide may comprise, consist of, or essentially consist of a polynucleotide sequence of SEQ ID NO: 2 or a polynucleotide sequence having 70% or more homology or identity thereto, but is not limited thereto. For example, the polynucleotide may consist of a base sequence having 70% or more, 80% or more, 90% or more, 95% or more, 96% or more, 97% or more, 98% or more, or 99% or more homology or identity to SEQ ID NO: 2, but is not limited thereto.

Further, the polynucleotide of the present disclosure may comprise a probe, for example, without limitation as long as it is a sequence capable of hybridizing with a complementary sequence to the entirety or part of the polynucleotide base sequence under stringent conditions. The term "stringent conditions" refers to conditions that enable specific hybridization between polynucleotides. These conditions are specifically described in the literature (such as J. Sambrook *et al., supra*). Examples of stringent conditions may be conditions in which polynucleotides having high homology or identity of 40% or more, specifically 90% or more, more specifically 95% or more, 96% or more, 97% or more, or 98% or more, or even more specifically 99% or more, hybridize with each other, but polynucleotides having low homology or identity do not hybridize with each other; or washing conditions of typical Southern hybridization, *i.e.,* conditions of washing once, or specifically two to three times, at a salt concentration and temperature corresponding to 1×SSC, 0.1% SDS at 60°C, specifically 0.1×SSC, 0.1% SDS at 60°C, or more specifically 0.1×SSC, 0.1% SDS at 68°C.

Hybridization requires that two nucleic acids have complementary sequences, although mismatches between bases may be possible depending on the stringency of hybridization. The term "complementary" is used to describe the relationship between nucleotide bases capable of hybridizing with each other. For example, with regard to DNA, adenine is complementary to thymine, and cytosine is complementary to guanine. Hence, the polynucleotide of the present disclosure may also include substantially similar nucleic acid sequences as well as isolated nucleic acid fragments that are complementary to the entire sequence.

Specifically, polynucleotides having homology or identity may be detected by employing hybridization conditions comprising a hybridization step at a Tm of 55°C and utilizing the above-described conditions. In addition, the Tm value may be 60°C, 63°C, or 65°C, but is not limited thereto, and may be appropriately adjusted by those skilled in the art.

The appropriate stringency for hybridizing a polynucleotide depends on the length and degree of complementarity of the polynucleotide, and the variables are well known in the relevant technical field (see J. Sambrook *et al., supra*).

In the present disclosure, the "vector" may comprise a DNA construct for inserting a polynucleotide encoding the SNQ2 protein of the present disclosure into a chromosome of a host, or a DNA construct comprising a base sequence of a polynucleotide encoding the SNQ2 protein that is operably linked to a suitable expression regulatory region (or expression control sequence) such that the desired polypeptide or protein may be expressed in a suitable host. The expression regulatory region may comprise a promoter capable of initiating transcription, any operator sequence for controlling such transcription, a sequence encoding a suitable mRNA ribosome binding site, and a sequence controlling termination of transcription and translation. The vector, after being transformed into a suitable host cell, may replicate or function independently of the host genome, or may be integrated into the genome itself.

The vector of the present disclosure may be an expression vector for expressing the SNQ2 protein of the present disclosure in a host cell (including microorganism), but is not limited thereto.

Further, the vector of the present disclosure may be an insertion vector for inserting a polynucleotide encoding the SNQ2 protein of the present disclosure into a chromosome, but is not limited thereto. The insertion of the polynucleotide into the chromosome may be performed by any method known in the art, for example, homologous recombination, but is not limited thereto. It may further comprise a selection marker for confirming the insertion into the chromosome. The selection marker is for selecting cells transformed with vectors, that is, for confirming the insertion of a desired nucleic acid molecule, and markers that confer selectable phenotypes such as drug resistance, auxotrophy, resistance to cytotoxic agents, or expression of surface polypeptides or proteins may be used. In an environment treated with a selective agent, only cells expressing the selection marker survive or exhibit other phenotypic traits, thereby enabling selection of transformed cells. The insertion vector may not comprise an origin of replication required for replication in transformed cells.

The vector used in the present disclosure is not particularly limited, and any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or a cosmid vector, and pDZ system, pBR system, pUC system, pBluescript II system, pGEM system, pTZ system, pCL system, pET system, *etc.* may be used as a plasmid vector. Specifically, pDZ, pDC, pDCM2, pACYC177, pACYC184, pCL, pECCG117, pUC19, pBR322, pMW118, pCC1BAC vectors, *etc.* may be used.

As used herein, the term "transformation" refers to the introduction of a vector comprising a polynucleotide encoding the SNQ2 protein of the present disclosure into a host cell, such that the protein encoded by the polynucleotide can be expressed in the host cell. The transformed polynucleotide may be inserted and located within a chromosome of the host cell or located outside the chromosome, as long as it can be expressed in the host cell. Further, the polynucleotide comprises a DNA and/or RNA encoding the protein. The polynucleotide may be introduced into a host cell in any form, as long as it can be expressed therein. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replication. Further, the polynucleotide may be introduced into a host cell in the form *per se* and be operably linked to a sequence required for expression in the host cell, but is not limited thereto.

In addition, as used herein, the term "operably linked" means that a polynucleotide encoding the SNQ2 protein of the present disclosure is functionally linked to a promoter sequence that initiates and mediates transcription of the polynucleotide.

The method of transforming a vector of the present disclosure comprises any method of introducing a nucleic acid into a cell, and may be performed by selecting a suitable standard technique known in the art depending on the host cell. For example, methods such as electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method may be used, but are not limited thereto.

As used herein, the term "microorganism" or "strain" includes both wild-type microorganisms or microorganisms that have undergone natural or artificial genetic modification. It may be a microorganism in which a specific mechanism is weakened or enhanced due to insertion of a foreign gene or enhancement or inactivation of activity of an endogenous gene, and may be the microorganism of the present disclosure comprising a genetic modification for the enhancement of SNQ or product production.

The microorganism of the present disclosure is a *Yarrowia lipolytica* microorganism having retinoid-producing ability. The *"Yarrowia lipolytica* microorganism having retinoid-producing ability" may be used interchangeably with "retinoid-producing *Yarrowia lipolytica* microorganism".

The microorganism of the present disclosure can produce retinoid without utilizing microbial cell disruption or using dodecane as a solvent, which are widely used for retinoid extraction.

The microorganism of the present disclosure may be a *Yarrowia lipolytica* microorganism with increased retinoid-producing ability and/or retinoid-excreting ability as a result of an enhanced activity of an SNQ2 protein.

In the microorganism of the present disclosure, the increase in retinoid-producing ability may be due to an increase in retinoid-excreting ability, but is not limited thereto.

The microorganism of the present disclosure may selectively excrete retinoid, but is not limited thereto.

In one embodiment, the microorganism of the present disclosure may selectively excrete retinoid among beta-carotene and retinoid.

The microorganism of the present disclosure may be a microorganism in which the activity of an endogenous SNQ2 protein is enhanced; a microorganism that naturally has an SNQ2 protein or retinoid-producing ability; a microorganism in which the SNQ2 protein of the present disclosure, which has an enhanced activity compared to an endogenous SNQ2 protein, is introduced into a parent strain lacking an SNQ2 protein or retinoid-producing ability; or a microorganism in which the SNQ2 protein of the present disclosure is further introduced into a parent strain having an SNQ2 protein or retinoid-producing ability.

In one example, the microorganism of the present disclosure is a cell or microorganism with enhanced activity of an SNQ2 protein, in which the activity is enhanced by transformation with a polynucleotide encoding the SNQ2 protein of the present disclosure; and according to the objects of the present disclosure, the microorganism of the present disclosure may encompass all microorganisms capable of producing retinoids by comprising the SNQ2 protein of the present disclosure.

The microorganism of the present disclosure may have increased retinoid-excreting ability compared to a *Yarrowia lipolytica* microorganism having retinoid-producing ability without enhanced activity of an SNQ2 protein.

For example, the microorganism of the present disclosure may be a recombinant strain with increased retinoid-producing ability and/or retinoid-excreting ability by having an enhanced activity of an SNQ2 protein resulting from further introducing a polynucleotide encoding the SNQ2 protein of the present disclosure into a natural wild-type microorganism, a microorganism having retinoid-producing ability, and/or a microorganism comprising an SNQ2 protein. The recombinant strain with increased retinoid-producing ability and/or retinoid-excreting ability may be a microorganism with increased retinoid-producing ability and/or retinoid-excreting ability compared to a natural wild-type microorganism, a microorganism in which the activity of the SNQ2 protein of the present disclosure has not been enhanced, or a microorganism that does not overexpress SNQ2 proteins, but is not limited thereto.

For example, the microorganism in which the SNQ2 protein of the present disclosure has not been enhanced, used as a reference strain for comparing whether the retinoid-producing ability and retinoid-excreting ability have increased, may be CC08-2050 (KCCM13294P, Ref. Park et al., Metabolic Engineering 2022;73:26-37), but is not limited thereto. The deposited strain name of the CJ2050 strain disclosed in the cited reference (Park et al., Metabolic Engineering 2022;73:26-37) is the CC08-2050 strain of the present disclosure, and thus, the CC08-2050 strain of the present disclosure and the CJ2050 strain are the same strain.

In one example, the recombinant strain with increased retinoid-producing ability and/or retinoid-excreting ability may have an increased retinoid-producing ability and/or retinoid-excreting ability compared to the retinoid-producing ability and/or retinoid-excreting ability of the parent strain before modification or a non-modified microorganism by about 0.001% or more or 0.01% or more, but is not limited thereto as long as there is a positive increase compared to the producing ability and/or retinoid-excreting ability of the parent strain before modification or the non-modified microorganism. The term "about" encompasses a range of ±0.5, ±0.4, ±0.3, ±0.2, ±0.1, *etc.,* and all values equivalent to or similar to the numerical value following the term "about", but is not limited thereto.

As used herein, the term "non-modified microorganism" does not exclude a strain comprising mutations that may occur naturally in a microorganism, and may refer to a wild-type microorganism or a natural microorganism *per se,* or a strain before a trait is altered by genetic variation due to natural or artificial factors. For example, the non-modified microorganism may refer to a strain in which the activity of the SNQ2 protein of the present disclosure is not enhanced or has not yet been enhanced. The term "non-modified microorganism" may be used interchangeably with "strain before modification", "microorganism before modification", "non-mutated strain", "non-modified strain", "non-mutated microorganism", or "reference microorganism".

The microorganism having retinoid-producing ability of the present disclosure may be a microorganism into which a polynucleotide encoding lycopene cyclase/phytoene synthase (crtYB) and phytoene desaturase (crtl) proteins has been introduced so as to enable a microorganism intrinsically lacking retinoid-producing ability to have retinoid-producing ability, or to further enhance the retinoid-producing ability of a microorganism having retinoid-producing ability, and thereby exhibit the activities of these proteins or have enhanced activities of these proteins. The lycopene cyclase/phytoene synthase or phytoene desaturase may be proteins derived from *Xanthophyllomyces dendrorhous,* but are not limited thereto as long as they are proteins that exhibit the same or similar activities. In one embodiment, the lycopene cyclase/phytoene synthase or phytoene desaturase may consist of or comprise the amino acid sequence of SEQ ID NO: 11 or SEQ ID NO: 13, respectively, but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the above amino acid sequences while exhibiting an activity corresponding to the lycopene cyclase/phytoene synthase or phytoene desaturase. Further, it is apparent that a protein with deletion, modification, substitution, or addition to a part thereof is also included in the lycopene cyclase/phytoene synthase or phytoene desaturase, as long as the protein has the aforementioned homology or identity and exhibits an activity corresponding to the lycopene cyclase/phytoene synthase or phytoene desaturase. Further, in one embodiment, the polynucleotide encoding the lycopene cyclase/phytoene synthase or phytoene desaturase may consist of or comprise the sequence of SEQ ID NO: 12 or SEQ ID NO: 14, respectively. In the polynucleotide, various modifications may be made in the coding region as long as the amino acid sequence is not changed in consideration of codon degeneracy or codons preferred in the microorganism of the present disclosure. Specifically, the polynucleotide may consist of or comprise a base sequence having 60% or more, 70% or more, 80% or more, 85% or more, 90% or more, 95% or more, 96% or more, 97% or more, 98% or more, and less than 100% homology or identity to the sequence of SEQ ID NO: 12 or SEQ ID NO: 14, but is not limited thereto.

Further, the microorganism of the present disclosure may be a microorganism into which a polynucleotide encoding geranylgeranyl pyrophosphate synthase (GGPPS) protein has been introduced so as to enable a microorganism intrinsically lacking retinoid-producing ability to have retinoid-producing ability, or to further enhance the retinoid-producing ability of a microorganism having retinoid-producing ability, and thereby exhibit geranylgeranyl pyrophosphate synthase activity or have enhanced geranylgeranyl pyrophosphate synthase activity. The geranylgeranyl pyrophosphate synthase may be a protein derived from *Haematococcus pluvialis,* but is not limited thereto as long as it is a protein that exhibits the same or similar activity. In one embodiment, the geranylgeranyl pyrophosphate synthase may consist of or comprise the amino acid sequence of SEQ ID NO: 15 but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the amino acid sequence of SEQ ID NO: 15 while exhibiting an activity corresponding to the geranylgeranyl pyrophosphate synthase. Further, it is apparent that a protein with deletion, modification, substitution, or addition to a part thereof is also included in the geranylgeranyl pyrophosphate synthase, as long as the protein has the aforementioned homology or identity and exhibits an activity corresponding to the geranylgeranyl pyrophosphate synthase. Further, in one embodiment, the polynucleotide encoding the geranylgeranyl pyrophosphate synthase may have or comprise the sequence of SEQ ID NO: 16. In the polynucleotide, various modifications may be made in the coding region as long as the amino acid sequence is not changed in consideration of codon degeneracy or codons preferred in the microorganism of the present disclosure. Specifically, the polynucleotide may consist of or comprise a base sequence having 60% or more, 70% or more, 80% or more, 85% or more, 90% or more, 95% or more, 96% or more, 97% or more, 98% or more, and less than 100% homology or identity to the sequence of SEQ ID NO: 16, but is not limited thereto.

Further, the microorganism of the present disclosure may be a microorganism into which a polynucleotide encoding beta-carotene 15,15'-oxygenase (BLH) protein has been introduced so as to enable a microorganism intrinsically lacking retinoid-producing ability to have retinoid-producing ability, or to further enhance the retinoid-producing ability of a microorganism having retinoid-producing ability, and thereby exhibit beta-carotene 15,15'-oxygenase activity or have enhanced beta-carotene 15,15'-oxygenase activity. The beta-carotene 15,15'-oxygenase may be a protein derived from uncultured marine bacterium 66A03, but is not limited thereto as long as it is a protein that exhibits the same or similar activity. In one embodiment, the beta-carotene 15,15'-oxygenase may consist of or comprise the amino acid sequence of SEQ ID NO: 17 but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the amino acid sequence of SEQ ID NO: 17 while exhibiting an activity corresponding to the beta-carotene 15,15'-oxygenase. Further, it is apparent that a protein with deletion, modification, substitution, or addition to a part thereof is also included in the beta-carotene 15,15'-oxygenase, as long as the protein has the aforementioned homology or identity and exhibits an activity corresponding to the beta-carotene 15,15'-oxygenase. Further, in one embodiment, the polynucleotide encoding the beta-carotene 15,15'-oxygenase may have or comprise the sequence of SEQ ID NO: 18. In the polynucleotide, various modifications may be made in the coding region as long as the amino acid sequence is not changed in consideration of codon degeneracy or codons preferred in the microorganism of the present disclosure. Specifically, the polynucleotide may consist of or comprise a base sequence having 60% or more, 70% or more, 80% or more, 85% or more, 90% or more, 95% or more, 96% or more, 97% or more, 98% or more, and less than 100% homology or identity to the sequence of SEQ ID NO: 18, but is not limited thereto.

In one embodiment, one of the microorganisms having retinoid-producing ability of the present disclosure, CC08-2050 (KCCM13294P), comprises: a lycopene cyclase/phytoene synthase comprising the amino acid sequence of SEQ ID NO: 11; a phytoene desaturase comprising the amino acid sequence of SEQ ID NO: 13; a geranylgeranyl pyrophosphate synthase comprising the amino acid sequence of SEQ ID NO: 15; and a beta-carotene 15,15'-oxygenase comprising the amino acid sequence of SEQ ID NO: 17.

As used herein, an "enhancement" in protein activity means that the activity of a protein is increased compared to its endogenous activity. The increase may be used interchangeably with terms such as "activation", "up-regulation", "overexpression", and "increase".

Herein, activation, enhancement, upregulation, overexpression, or increase may include both exhibiting an activity not originally possessed, or exhibiting an improved activity compared to its endogenous activity or activity before modification.

Accordingly, a microorganism expressing an SNQ2 protein or a microorganism with an introduced SNQ2 protein may also be referred to as a microorganism with enhanced activity of an SNQ2 protein compared to its endogenous activity.

The "endogenous activity" refers to the activity of a specific protein originally possessed by a parent strain before transformation or a non-modified microorganism, when the trait is altered by genetic variation due to natural or artificial factors. This term may be used interchangeably with "activity before modification". The "enhancement", "up-regulation", "overexpression", or "increase" in activity of a protein compared to its endogenous activity means an improvement compared to the activity and/or concentration (expression level) of a specific protein originally possessed by the parent strain before transformation or a non-modified microorganism.

The enhancement can be achieved by introducing a foreign protein (however, in the case of enhancement of the activity of an SNQ2 protein, the introduction refers to the introduction of a protein derived from *Yarrowia lipolytica* exhibiting the activity of SNQ2; a protein comprising the amino acid sequence of SEQ ID NO: 1 or an amino acid sequence having at least 70% homology thereto; and/or a polynucleotide encoding the same) or by enhancing the activity and/or concentration (expression level) of an endogenous protein. Whether activity of a protein has been enhanced may be confirmed from the increase in the activity level of the protein, its expression level, or the amount of product excreted from the protein.

The enhancement in the activity of a protein can employ various methods well known in the art, and is not limited as long as the activity of the desired protein can be enhanced compared to that of the microorganism before modification. Specifically, genetic engineering and/or protein engineering well known to those skilled in the art as routine methods in molecular biology may be used, but the method is not limited thereto (*e.g.,* Sitnicka et al. Functional Analysis of Genes. Advances in Cell Biology. 2010, Vol. 2. 1-16, Sambrook et al. Molecular Cloning 2012, *etc*.).

Specifically, the enhancement in the activity of a protein of the present disclosure may be:
1) increase in the intracellular copy number of a polynucleotide encoding the protein;
2) replacement of a gene expression regulatory region on a chromosome encoding the protein with a highly active sequence;
3) modification of a base sequence encoding a start codon or 5'-UTR region of a gene transcript encoding the protein;
4) modification of an amino acid sequence of the protein to enhance the activity of the protein;
5) modification of a polynucleotide sequence encoding the protein to enhance the activity of the protein (*e.g.,* modification of a polynucleotide sequence of the gene encoding the protein, such that the gene encodes a protein modified to have enhanced activity);
6) introduction of a foreign protein exhibiting the activity of the protein, or a foreign polynucleotide encoding the same (however, in the case of enhancement of the activity of an SNQ2 protein, the introduction refers to the introduction of a protein derived from *Yarrowia lipolytica* exhibiting the activity of SNQ2; a protein comprising the amino acid sequence of SEQ ID NO: 1 or an amino acid sequence having at least 70% homology thereto; and/or a polynucleotide encoding the same);
7) codon optimization of a polynucleotide encoding the protein;
8) selection and modification or chemical modification of exposed regions of the protein through an analysis of tertiary structure thereof;
9) regulation of the cellular localization of the protein (polypeptide); or
10) a combination of two or more selected from 1) to 9), but is not particularly limited thereto.

For example,
The 1) increase in the intracellular copy number of a polynucleotide encoding the protein (polypeptide) may involve introducing a vector comprising a polynucleotide encoding the protein (polypeptide) operably linked to an appropriate regulatory sequence into a host cell (microorganism). Alternatively, the increase may involve introducing one copy or two or more copies of the polynucleotide encoding the protein (polypeptide) operably linked to an appropriate regulatory sequence into a chromosome of a host cell (microorganism). The introduction into a chromosome may be performed by introducing into a host cell (microorganism) a vector capable of inserting the polynucleotide into a chromosome of the host cell (microorganism), but is not limited thereto. The vector is as described above. The regulatory sequence may be a native sequence (of the same origin) or a foreign sequence (derived from a different gene) with respect to the polynucleotide sequence encoding the protein, a varied sequence thereof, or a different artificial sequence, and may induce the expression of the polynucleotide in the host cell (microorganism).

In one embodiment, in the enhancement in the activity of an SNQ2 protein of the present disclosure, the regulatory sequence of the gene encoding the SNQ2 protein may be a TEF promoter, but is not limited thereto.

The 2) replacement of a gene expression regulatory region (or expression control sequence) on a chromosome encoding the protein (polypeptide) with a highly active sequence may involve, for example, introducing a modification in a sequence by deletion, insertion, substitution, or a combination thereof such that the expression-inducing activity of the expression regulatory region is further enhanced, or a replacement with a sequence exhibiting a stronger activity. The expression regulatory region may comprise, but is not particularly limited to, a promoter, an operator sequence, a sequence encoding a ribosome binding site, a sequence controlling the termination of transcription and translation, *etc.* In one example, it may involve replacing the original promoter with a strong promoter, but is not limited thereto.

Examples of known strong promoters include cj1 to cj7 promoters (U.S. Patent No. US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (U.S. Patent No. US 10584338 B2), an O2 promoter (U.S. Patent No. US 10273491 B2), a tkt promoter, a yccA promoter, a TEF promoter, *etc.,* but are not limited thereto.

In one embodiment, the native promoter of a gene encoding SNQ2 in the *Yarrowia lipolytica* microorganism of the present disclosure may be replaced by a TEF promoter, but is not limited thereto.

The 3) modification of a base sequence encoding a start codon or 5'-UTR region of a gene encoding the protein (polypeptide) may involve, for example, substituting with another start codon with a higher protein (polypeptide) expression rate as compared to an endogenous start codon, or modifying to code for a ribosome binding site (RBS) sequence with a higher protein (polypeptide) expression rate as compared to an endogenous RBS sequence, but is not limited thereto.

The 4) and 5) modification of the amino acid sequence or the polynucleotide sequence of the protein (polypeptide) may involve introducing a modification in the sequence by deletion, insertion, substitution, or a combination thereof in the amino acid sequence of the protein (polypeptide) or the polynucleotide sequence encoding the protein (polypeptide), or replacing with an amino acid sequence or polynucleotide sequence modified to exhibit increased activity, but is not limited thereto. The replacement may, for example, be performed by inserting a polynucleotide into a chromosome through homologous recombination, but is not limited thereto.

The 6) introduction of a foreign polynucleotide exhibiting the activity of the protein (polypeptide) may involve introducing a foreign polynucleotide into a host cell (microorganism), the foreign polynucleotide encoding a protein (polypeptide) exhibiting an activity identical or similar to that of the protein (polypeptide). The foreign polynucleotide is not limited in its origin or sequence as long as it exhibits an activity identical or similar to that of the protein (polypeptide). The method used for the introduction may be performed by appropriately selecting a known transformation method by those skilled in the art. As the introduced polynucleotide is expressed in the host cell, the protein (polypeptide) may be produced, and the activity thereof may be increased.

The 7) codon optimization of a polynucleotide encoding the protein (polypeptide) may be a codon optimization such that the transcription or translation of an endogenous polynucleotide is increased in a host cell (microorganism), or such that the transcription or translation of a foreign polynucleotide is optimized in a host cell (microorganism).

The 8) selection and modification or chemical modification of exposed regions of the protein (polypeptide) through an analysis of tertiary structure thereof may involve, for example, determining a template protein candidate based on the degree of similarity of the sequence by comparing the sequence information of a protein (polypeptide) to be analyzed with a database storing the sequence information of known proteins, confirming the structure based thereon, selecting an exposed site to be modified or chemically modified, and modifying or chemically modifying the same.

The 9) regulation of the cellular localization of the protein (polypeptide) may involve targeting the protein (polypeptide) to a specific cellular organelle or a specific intracellular space. For example, it may involve targeting a protein (polypeptide) to the periplasm or cytoplasm by adding or removing a leader sequence that functions in the targeting of the protein (polypeptide), but is not limited thereto.

Such increase in the protein (polypeptide) activity may result in an increase in the activity or concentration of the corresponding protein (polypeptide) as compared to the activity or concentration of the protein (polypeptide) expressed in a wild-type host cell (microorganism) or a host cell (microorganism) before modification, or an increase in the amount of product resulting from the activity of the protein (polypeptide), but is not limited thereto.

In the present disclosure, "retinoid" refers to a group of compounds chemically belonging to the vitamin A family or chemically related thereto.

In one embodiment, the retinoid may be any one selected from the group consisting of retinol, retinal, retinoic acid, and retinyl ester, but is not limited thereto.

In one embodiment, retinol can be converted into other retinoids (*e.g.,* retinal, retinoic acid, retinyl ester, *etc*.) or carotenoid compounds by methods known in the art.

Another aspect of the present disclosure provides a method for producing retinoid, comprising culturing the microorganism of the present disclosure in a medium.

The microorganism, retinoid, *etc.* are as described in another aspect.

As used herein, the term "cultivation" means growing the *Yarrowia lipolytica* microorganism of the present disclosure under appropriately controlled environmental conditions. The cultivation process of the present disclosure may be performed according to a suitable medium and cultivation conditions known in the art. Such a cultivation process may be easily adjusted and used by those skilled in the art according to the selected strain. Specifically, the cultivation may be a batch type, continuous type, and/or fed-batch type, but is not limited thereto.

The *Yarrowia lipolytica* microorganism of the present disclosure may be cultured in a common medium containing appropriate carbon sources, nitrogen sources, phosphorus sources, inorganic compounds, amino acids and/or vitamins, *etc.,* while controlling the temperature, pH, *etc.* under aerobic conditions.

In the present disclosure, the carbon sources may comprise carbohydrates such as glucose, saccharose, lactose, fructose, sucrose, maltose, *etc.;* sugar alcohols such as mannitol, sorbitol, *etc*.; organic acids such as pyruvic acid, lactic acid, citric acid, *etc.;* and amino acids such as glutamic acid, methionine, lysine, *etc.* Further, natural organic nutrient sources such as starch hydrolysate, molasses, blackstrap molasses, rice bran, cassava, sugarcane residue, and corn steep liquor may be used. Specifically, carbohydrates such as glucose and sterilized pretreated molasses (*i.e.,* molasses converted to reducing sugar) may be used, and appropriate amounts of other carbon sources may be used in various manners without limitation. These carbon sources may be used alone or in combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extracts, yeast extracts, malt extracts, corn steep liquor, casein hydrolysates, fish or decomposition products thereof, defatted soybean cake or degradation products thereof, *etc.* may be used. These nitrogen sources may be used alone or in a combination of two or more thereof, but are not limited thereto.

The phosphorus sources may comprise monopotassium phosphate, dipotassium phosphate, or sodium-containing salts corresponding thereto. As for inorganic compounds, sodium chloride, calcium chloride, iron chloride, magnesium sulfate, iron sulfate, manganese sulfate, calcium carbonate, *etc.* may be used, and in addition, amino acids, vitamins, and/or suitable precursors may be included. These constituents or precursors may be added to the medium in a batch or continuous manner, but are not limited thereto.

During the cultivation of the *Yarrowia lipolytica* microorganism of the present disclosure, the pH of the medium may be adjusted by adding compounds such as ammonium hydroxide, potassium hydroxide, ammonia, phosphoric acid, sulfuric acid, *etc.* to the medium in a suitable manner. Further, during the cultivation, foaming may be suppressed by using an antifoaming agent such as fatty acid polyglycol ester. Additionally, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but are not limited thereto.

Further, the culture medium may contain metal salts such as magnesium sulfate or iron sulfate necessary for growth. Lastly, in addition to the aforementioned substances, essential growth substances such as amino acids and vitamins may be used. Additionally, suitable precursors may be used in the culture medium. The aforementioned raw materials may be added to the culture in a batch or continuous manner by a suitable method during the cultivation process, but are not limited thereto.

During the cultivation of the microorganism of the present disclosure, the pH of the culture may be adjusted by adding compounds such as ammonium hydroxide, potassium hydroxide, ammonia, phosphoric acid, sulfuric acid, *etc.* to the culture in a suitable manner. Further, during the cultivation, foaming may be suppressed by using an antifoaming agent such as fatty acid polyglycol ester. Additionally, oxygen or oxygen-containing gas may be injected into the culture in order to maintain the aerobic state of the culture, or gas may not be injected or nitrogen, hydrogen, or carbon dioxide gas may be injected in order to maintain the anaerobic and microaerobic states, but are not limited thereto.

In the cultivation of the present disclosure, the cultivation temperature may be maintained at 20°C to 35°C, specifically 25°C to 35°C. The cultivation period may be continued until the amount of the useful substance is obtained, and may be about 10 hours to 160 hours, about 20 hours to 130 hours, about 24 hours to 120 hours, about 36 hours to 120 hours, about 48 hours to 120 hours, about 48 hours or more, or about 48 hours, about 72 hours, or about 120 hours, but is not limited thereto.

The method for producing retinoid of the present disclosure may further comprise recovering retinoid from the microorganism or the medium.

The desired retinoid may be recovered from the medium by using an appropriate method known in the art according to the cultivation method of the microorganism of the present disclosure, for example, a batch, continuous, or fed-batch type cultivation method. For example, centrifugation, filtration, treatment with a crystallized protein precipitating agent (salting-out), extraction, sonication, ultrafiltration, dialysis, various types of chromatography such as molecular sieve chromatography (gel filtration), adsorption chromatography, ion exchange chromatography, affinity chromatography, *etc.,* HPLC, and a combination of these methods may be used, but are not limited thereto.

The method for producing retinoid may further comprise a purification process. The purification process may be performed by using an appropriate method known in the art.

In one embodiment, the method for producing retinoid of the present disclosure uses a microorganism having retinoid-excreting ability with enhanced activity of an SNQ2 protein, and therefore, the present disclosure can produce retinoid without utilizing microbial cell disruption or using dodecane as a solvent, which are widely used for retinoid extraction; however, the method is not limited thereto.

The method for producing retinoid of the present disclosure may further comprise conversion of retinol expressed by the microorganism of the present disclosure into a retinoid other than retinol. With regard to the method for producing retinoid of the present disclosure, the conversion may be further included after the cultivation or the recovery. The conversion may be performed by using an appropriate method known in the art. For example, the conversion may be performed using retinol acyltransferase, but is not limited thereto.

In one embodiment, the retinoid may be any one selected from the group consisting of retinal, retinoic acid, and retinyl ester, but is not limited thereto as long as it is included in retinoids.

Another aspect of the present disclosure provides a method for preparing a *Yarrowia lipolytica* microorganism having retinoid-producing ability, comprising enhancing the activity of an SNQ2 protein in the *Yarrowia lipolytica* microorganism having retinoid-producing ability.

Another aspect of the present disclosure provides a method for increasing retinoid excretion, comprising enhancing the activity of an SNQ2 protein in the *Yarrowia lipolytica* microorganism having retinoid-producing ability.

The method for increasing retinoid excretion may be a method for increasing retinoid excretion in a *Yarrowia lipolytica* microorganism having retinoid-producing ability.

The step of enhancing the activity of an SNQ2 protein may involve modifying a *Yarrowia lipolytica* microorganism to express SNQ2 protein, as described in other aspects.

The SNQ2 protein, the enhancement in activity thereof, the microorganism, the retinoid, *etc.,* are as described in other aspects.

Another aspect of the present disclosure provides a composition for producing retinoid, comprising one or more of a *Yarrowia lipolytica* microorganism having retinoid-producing ability with enhanced activity of an SNQ2 protein, and a culture thereof.

The composition of the present disclosure may further comprise any buffer and/or appropriate excipient that is commonly used in compositions for retinoid production. Such excipients may be, for example, a preservative, a wetting agent, a dispersing agent, a suspending agent, a buffering agent, a stabilizing agent, or an isotonic agent, *etc.,* but are not limited thereto.

The SNQ2 protein, the enhancement in activity thereof, the microorganism, the retinoid, *etc.,* are as described in other aspects.

Another aspect of the present disclosure provides a use of the SNQ2 protein of the present disclosure for increasing retinoid production.

Another aspect of the present disclosure provides a use of the *Yarrowia lipolytica* microorganism with enhanced activity of the SNQ2 protein of the present disclosure for retinoid production.

The SNQ2 protein, the enhancement in activity thereof, the microorganism, the retinoid, *etc.,* are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Deletion of DPP1 in a retinoid-producing Y. lipolytica strain

In the retinoid-producing *Yarrowia lipolytica* strain KCCM13294P, the DPP1 (YALI0C11297g) gene in the genome was deleted to secure a site for foreign gene insertion. To this end, the ORF sequence (SEQ ID NO: 19) of DPP1 (YALI0C11297g) was obtained based on the nucleotide sequence registered in KEGG (Kyoto Encyclopedia of Genes and Genomes). Additionally, a DPP1 (YALI0C11297g) deletion cassette was constructed using the URA3 gene (SEQ ID NO: 22) of *Y. lipolytica* as a selection marker.

To this end, the genomic DNA of KCCM13294P was used as a template and PCR was performed using the primers of SEQ ID NO: 24 and SEQ ID NO: 25, SEQ ID NO: 26 and SEQ ID NO: 27, SEQ ID NO: 28 and SEQ ID NO: 29, and SEQ ID NO: 30 and SEQ ID NO: 31 on the left homologous region, URA3, repeat region, and right homologous region fragments, respectively, as shown in Table 1. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the KCCM13294P strain using the heat shock method (*D*.-C. Chen et al., Appl Microbiol Biotechnol, 1997), and colonies formed on a solid medium lacking uracil (YLMM1) were obtained. The colonies, for which insertion of the cassette into the genome was confirmed using primers of SEQ ID NO: 32 and SEQ ID NO: 33, were cultured on 5-FOA solid medium at 30°C for 3 days, and by obtaining colonies grown on the 5-FOA solid medium, the URA3 marker was recovered.

The final DPP1-deleted strain thus obtained was named CC08-2373.

**[Table 1]**

| SEQ ID NO | Sequence (5'- 3') | PCR Product |
|---|---|---|
| 24 | AGGTCTTCTGGAACTATTATGTAAT | Homology left arm |
| 25 | ATGACGAGTCAGACAGGAGGCATCTTTAGCTGTGTTGA AAACAG | |
| 26 | CTGTTTTCAACACAGCTAAAGATGCCTCCTGTCTGACTC GTCAT | URA3 |
| 27 | AGGTCTTTTCTCATAAGCCGACTGGTGGTATTGTGACTG GGGAT | |
| 28 | ATCCCCAGTCACAATACCACCAGTCGGCTTATGAGAAAA GACCT | Repeat region |
| 29 | GATGTATTAACGACAGATTTCATCTTTAGCTGTGTTGAAA ACAG | |
| 30 | CTGTTTTCAACACAGCTAAAGATGAAATCTGTCGTTAATA CATC | Homology right arm |
| 31 | CCTGAAAAAAAAACACCTACTCAGC | |
| 32 | GGACATCATCACGTTCAGCTTATCC | Forward |
| 33 | ACCATACAATCGTTTCTGACCAATC | Reverse |

The aforementioned YLMM1 and 5-FOA media used had the following compositions:
*<Yarrowia lipolytica* Minimal Medium 1 (YLMM1)>
   Glucose 20 g/L, Yeast nitrogen base without amino acids 6.7 g/L, Yeast Synthetic Drop-out Medium Supplements without uracil 2 g/L, Agar 15 g/L
<5-Fluoroorotic Acid (5-FOA) Medium>
   Glucose 20 g/L, Yeast nitrogen base without amino acids 6.7 g/L, Yeast Synthetic Drop-out Medium Supplements without uracil 2 g/L, Uracil 50 µg/mL, 5-Fluoroorotic acid (5-FOA) 1 g/L, Agar 15 g/L

### Example 2: Enhancement of SNQ2 in a retinoid-producing Y. lipolytica strain

Based on the CC08-2373 strain constructed in Example 1, the native promoter of SNQ2 was replaced with a TEF promoter to enhance the expression of endogenous SNQ2. To this end, the ORF sequence (SEQ ID NO: 2) and promoter region sequence (SEQ ID NO: 20) of SNQ2 (YALI0F17996g) were obtained based on the nucleotide sequence registered in KEGG (Kyoto Encyclopedia of Genes and Genomes). Additionally, an SNQ2 enhanced cassette was constructed using TEF promoter (SEQ ID NO: 21) and the URA3 gene (SEQ ID NO: 22) of *Y. lipolytica* as a selection marker.

To this end, the genomic DNA of CC08-2373 was used as a template and PCR was performed using the primers of SEQ ID NO: 34 and SEQ ID NO: 35, SEQ ID NO: 36 and SEQ ID NO: 37, SEQ ID NO: 38 and SEQ ID NO: 39, SEQ ID NO: 40 and SEQ ID NO: 41, and SEQ ID NO: 42 and SEQ ID NO: 43 on the left homologous region, TEF promoter, URA3, repeat region, and right homologous region fragments, respectively, as shown in Table 2. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR.

**[Table 2]**

| SEQ ID NO | Sequence (5'- 3') | PCR Product |
|---|---|---|
| 34 | ATTCTCATCAGGTAGAGCCACCCACCTTGG | Homology left arm |
| 35 | ATGCGCCGCCAACCCGGTCTCTGGTGCTTGGAGTGGT GATATGT | |
| 36 | ACATATCACCACTCCAAGCACCAGAGACCGGGTTGGCG GCGCAT | TEF promoter |
| 37 | ATGACGAGTCAGACAGGAGGCATTTGAATGATTCTTATA CTCAG | |
| 38 | CTGAGTATAAGAATCATTCAAATGCCTCCTGTCTGACTC GTCAT | URA3 |
| 39 | ATGCGCCGCCAACCCGGTCTCTTGGTGGTATTGTGACT GGGGAT | |
| 40 | ATCCCCAGTCACAATACCACCAAGAGACCGGGTTGGCG GCGCAT | Repeat region |
| 41 | GATCGATAGGGCCGTTGTCCATTTTGAATGATTCTTATAC TCAG | |
| 42 | CTGAGTATAAGAATCATTCAAAATGGACAACGGCCCTAT CGATC | Homology right arm |
| 43 | AAATGGAAGCTCGGGTAGCCAGGGCCTCTG | |
| 44 | GATCGGGTCGGAAAGTGAGATAAA | Forward |
| 45 | TGGGTGTTGAAGACGCCAATGTTC | Reverse |

The cassette thus constructed was introduced into the CC08-2373 strain using the heat shock method *(*D.-C. Chen et al., Appl Microbiol Biotechnol, 1997), and colonies formed on a solid medium lacking uracil (YLMM1; same composition as YLMM1 medium in Example 1) were obtained. The colonies, for which insertion of the cassette into the genome was confirmed using primers of SEQ ID NO: 44 and SEQ ID NO: 45, were cultured on 5-FOA solid medium (same composition as 5-FOA medium in Example 1) at 30°C for 3 days, and by obtaining colonies grown on the 5-FOA solid medium, the URA3 marker was recovered. The final SNQ2-enhanced strain thus obtained was named CC08-2374.

### Example 3: Enhancement of S.cerevisiae-derived PDR10 in a retinoid-producing Y. lipolytica strain

Based on the CC08-2373 strain constructed in Example 1, PDR10 (hereinafter referred to as Sc.PDR10) was enhanced. To this end, the polynucleotide sequence of *S.cerevisiae*-derived PDR10 obtained based on the sequence (GenBank: KAG2512237.1) registered in NCBI (National Center for Biotechnology Information Search database) was codon-optimized for *Y. lipolytica* through http://genscript.com. Further, a gene thereof was synthesized (SEQ ID NO: 4) through Macrogen, Inc., and for its expression, a cassette was designed to be inserted at the DPP1 gene locus using TEF promoter (SEQ ID NO: 21) as the promoter, TDH3 terminator (SEQ ID NO: 23) as the terminator, and URA3 gene (SEQ ID NO: 22) as the selection marker.

To this end, the synthesized Sc.PDR10 gene and the genomic DNA of CC08-2373 were used as template, and PCR was performed using the primers of SEQ ID NO: 46 and SEQ ID NO: 47, SEQ ID NO: 48 and SEQ ID NO: 49, SEQ ID NO: 50 and SEQ ID NO: 51, SEQ ID NO: 52 and SEQ ID NO: 53, SEQ ID NO: 54 and SEQ ID NO: 55, SEQ ID NO: 56 and SEQ ID NO: 57, and SEQ ID NO: 58 and SEQ ID NO: 59 on the left homologous region, TEF promoter, Sc.PDR10 ORF, TDH3 terminator, URA3, repeat region, and right homologous region fragments, respectively, as shown in Table 3. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR.

**[Table 3]**

| SEQ ID NO | Sequence (5'- 3') | PCR Product |
|---|---|---|
| 46 | AGGTCTTCTGGAACTATTATGTAAT | Homology left arm |
| 47 | ATGCGCCGCCAACCCGGTCTCTTCTTTAGCTGTGTTGA AAACAG | |
| 48 | CTGTTTTCAACACAGCTAAAGAAGAGACCGGGTTGGCG GCGCAT | TEF promoter |
| 49 | AACTTGAGGGCGCTTGCAACATTTTGAATGATTCTTATAC TCAG | |
| 50 | CTGAGTATAAGAATCATTCAAAATGTTGCAAGCGCCCTC AAGTT | Sc.PDR10 |
| 51 | TTCGCTCTTGATCTTCGGATAGTTATTTCTTTAATTTTTTG CTT | |
| 52 | AAGCAAAAAATTAAAGAAATAACTATCCGAAGATCAAGAG CGAA | TDH3 terminator |
| 53 | ATGACGAGTCAGACAGGAGGCAGTCTTGGAACGGTGAA AAAGCC | |
| 54 | GGCTTTTTCACCGTTCCAAGACTGCCTCCTGTCTGACTC GTCAT | URA3 |
| 55 | TTCGCTCTTGATCTTCGGATAGTGGTGGTATTGTGACTG GGGAT | |
| 56 | ATCCCCAGTCACAATACCACCACTATCCGAAGATCAAGA GCGAA | Repeat region |
| 57 | GATGTATTAACGACAGATTTCAGTCTTGGAACGGTGAAA AAGCC | |
| 58 | GGCTTTTTCACCGTTCCAAGACTGAAATCTGTCGTTAAT ACATC | Homology right arm |
| 59 | CCTGAAAAAAAAACACCTACTCAGC | |
| 32 | GGACATCATCACGTTCAGCTTATCC | Forward |
| 33 | ACCATACAATCGTTTCTGACCAATC | Reverse |

The cassette thus constructed was introduced into the CC08-2373 strain using the heat shock method (D.-C. Chen et al., Appl Microbiol Biotechnol, 1997), and colonies formed on a solid medium lacking uracil (YLMM1; same composition as YLMM1 medium in Example 1) were obtained. The colonies, for which insertion of the cassette into the genome was confirmed using primers of SEQ ID NO: 32 and SEQ ID NO: 33, were cultured on 5-FOA solid medium (same composition as 5-FOA medium in Example 1) at 30°C for 3 days, and by obtaining colonies grown on the 5-FOA solid medium, the URA3 marker was recovered. The final Sc.PDR10-expressing strain thus obtained was named CC08-2488.

### Example 4: Expression of S.cerevisiae-derived SNQ2 in a retinoid-producing Y. lipolytica strain

Based on the CC08-2373 strain constructed in Example 1, *S.cerevisiae-*derived SNQ2 (hereinafter referred to as Sc.SNQ2) was expressed. To this end, the polynucleotide sequence of *S.cerevisiae*-derived SNQ2 obtained based on the sequence (NCBI Reference Sequence: NP_010294.1) registered in NCBI (National Center for Biotechnology Information Search database) was codon-optimized for *Y. lipolytica* through http://genscript.com. Further, a gene thereof was synthesized (SEQ ID NO: 6) through Macrogen, Inc., and for its expression, a cassette was designed to be inserted at the DPP1 gene locus using TEF promoter (SEQ ID NO: 21) as the promoter, TDH3 terminator (SEQ ID NO: 23) as the terminator, and URA3 gene (SEQ ID NO: 22) as the selection marker.

To this end, the synthesized Sc.SNQ2 gene and the genomic DNA of CC08-2373 were used as template, and PCR was performed using the primers of SEQ ID NO: 46 and SEQ ID NO: 47, SEQ ID NO: 48 and SEQ ID NO: 60, SEQ ID NO: 61 and SEQ ID NO: 62, SEQ ID NO: 63 and SEQ ID NO: 53, SEQ ID NO: 54 and SEQ ID NO: 55, SEQ ID NO: 56 and SEQ ID NO: 57, and SEQ ID NO: 58 and SEQ ID NO: 59 on the left homologous region, TEF promoter, Sc.SNQ2 ORF, TDH3 terminator, URA3, repeat region, and right homologous region fragments, respectively, as shown in Table 4. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR.

**[Table 4]**

| SEQ ID NO | Sequence (5'- 3') | PCR Product |
|---|---|---|
| 46 | AGGTCTTCTGGAACTATTATGTAAT | Homology left arm |
| 47 | ATGCGCCGCCAACCCGGTCTCTTCTTTAGCTGTGTTGA AAACAG | |
| 48 | CTGTTTTCAACACAGCTAAAGAAGAGACCGGGTTGGCG | TEF promoter |
| | GCGCAT | |
| 60 | GCGTGCTTTTGATATTGCTCATTTTGAATGATTCTTATACT CAG | |
| 61 | CTGAGTATAAGAATCATTCAAAATGAGCAATATCAAAAGC ACGC | Sc.SNQ2 |
| 62 | TTCGCTCTTGATCTTCGGATAGTTACTGCTTCTTTTTCCT TATG | |
| 63 | CATAAGGAAAAAGAAGCAGTAACTATCCGAAGATCAAGA GCGAA | TDH3 terminator |
| 53 | ATGACGAGTCAGACAGGAGGCAGTCTTGGAACGGTGAA AAAGCC | |
| 54 | GGCTTTTTCACCGTTCCAAGACTGCCTCCTGTCTGACTC GTCAT | URA3 |
| 55 | TTCGCTCTTGATCTTCGGATAGTGGTGGTATTGTGACTG GGGAT | |
| 56 | ATCCCCAGTCACAATACCACCACTATCCGAAGATCAAGA GCGAA | Repeat region |
| 57 | GATGTATTAACGACAGATTTCAGTCTTGGAACGGTGAAA AAGCC | |
| 58 | GGCTTTTTCACCGTTCCAAGACTGAAATCTGTCGTTAAT ACATC | Homology right arm |
| 59 | CCTGAAAAAAAAACACCTACTCAGC | |
| 32 | GGACATCATCACGTTCAGCTTATCC | Forward |
| 33 | ACCATACAATCGTTTCTGACCAATC | Reverse |

The cassette thus constructed was introduced into the CC08-2373 strain using the heat shock method (D.-C. Chen et al., Appl Microbiol Biotechnol, 1997), and colonies formed on a solid medium lacking uracil (YLMM1; same composition as YLMM1 medium in Example 1) were obtained. The colonies, for which insertion of the cassette into the genome was confirmed using primers of SEQ ID NO: 32 and SEQ ID NO: 33, were cultured on 5-FOA solid medium (same composition as 5-FOA medium in Example 1) at 30°C for 3 days, and by obtaining colonies grown on the 5-FOA solid medium, the URA3 marker was recovered. The final Sc.SNQ2-expressing strain thus obtained was named CC08-2492.

### Example 5: Expression of Escherichia coli-derived MsbA in a retinoid-producing Y. lipolytica strain

Based on the CC08-2373 strain constructed in Example 1, MsbA derived from *Escherichia coli* str. K-12 substr. MG1655 (hereinafter referred to as Ec.MsbA) was expressed. To this end, the polynucleotide sequence of *E. coli*-derived MsbA obtained based on the sequence (GenBank: AAC74000.1) registered in NCBI (National Center for Biotechnology Information Search database) was codon-optimized for *Y. lipolytica* through http://genscript.com. Further, a gene thereof was synthesized (SEQ ID NO: 8) through Macrogen, Inc., and for its expression, a cassette was designed to be inserted at the DPP1 gene locus using TEF promoter (SEQ ID NO: 21) as the promoter, TDH3 terminator (SEQ ID NO: 23) as the terminator, and URA3 gene (SEQ ID NO: 22) as the selection marker.

To this end, the synthesized Ec.MsbA gene and the genomic DNA of CC08-2373 were used as template, and PCR was performed using the primers of SEQ ID NO: 46 and SEQ ID NO: 47, SEQ ID NO: 48 and SEQ ID NO: 64, SEQ ID NO: 65 and SEQ ID NO: 66, SEQ ID NO: 67 and SEQ ID NO: 53, SEQ ID NO: 54 and SEQ ID NO: 55, SEQ ID NO: 56 and SEQ ID NO: 57, and SEQ ID NO: 58 and SEQ ID NO: 59 on the left homologous region, TEF promoter, Ec.MsbA ORF, TDH3 terminator, URA3, repeat region, and right homologous region fragments, respectively, as shown in Table 5. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR.

**[Table 5]**

| SEQ ID NO | Sequence (5'- 3') | PCR Product |
|---|---|---|
| 46 | AGGTCTTCTGGAACTATTATGTAAT | Homology left arm |
| 47 | ATGCGCCGCCAACCCGGTCTCTTCTTTAGCTGTGTTGA AAACAG | |
| 48 | CTGTTTTCAACACAGCTAAAGAAGAGACCGGGTTGGCG GCGCAT | TEF promoter |
| 64 | ACAGGTCCTTGTCATTGTGCATTTTGAATGATTCTTATAC TCAG | |
| 65 | CTGAGTATAAGAATCATTCAAAATGCACAATGACAAGGA CCTGT | Ec.MsbA |
| 66 | TTCGCTCTTGATCTTCGGATAGTCATTGGCCAAACTGCA TCTTG | |
| 67 | CAAGATGCAGTTTGGCCAATGACTATCCGAAGATCAAGA GCGAA | TDH3 terminator |
| 53 | ATGACGAGTCAGACAGGAGGCAGTCTTGGAACGGTGAA AAAGCC | |
| 54 | GGCTTTTTCACCGTTCCAAGACTGCCTCCTGTCTGACTC GTCAT | URA3 |
| 55 | TTCGCTCTTGATCTTCGGATAGTGGTGGTATTGTGACTG | |
| | GGGAT | |
| 56 | ATCCCCAGTCACAATACCACCACTATCCGAAGATCAAGA GCGAA | Repeat region |
| 57 | GATGTATTAACGACAGATTTCAGTCTTGGAACGGTGAAA AAGCC | |
| 58 | GGCTTTTTCACCGTTCCAAGACTGAAATCTGTCGTTAAT ACATC | Homology right arm |
| 59 | CCTGAAAAAAAAACACCTACTCAGC | |
| 32 | GGACATCATCACGTTCAGCTTATCC | Forward |
| 33 | ACCATACAATCGTTTCTGACCAATC | Reverse |

The cassette thus constructed was introduced into the CC08-2373 strain using the heat shock method (D.-C. Chen et al., Appl Microbiol Biotechnol, 1997), and colonies formed on a solid medium lacking uracil (YLMM1; same composition as YLMM1 medium in Example 1) were obtained. The colonies, for which insertion of the cassette into the genome was confirmed using primers of SEQ ID NO: 32 and SEQ ID NO: 33, were cultured on 5-FOA solid medium (same composition as 5-FOA medium in Example 1) at 30°C for 3 days, and by obtaining colonies grown on the 5-FOA solid medium, the URA3 marker was recovered. The final Ec.MsbA-expressing strain thus obtained was named CC08-2376.

### Example 6: Expression of Salmonella enterica-derived MsbA in a retinoid-producing Y. lipolytica strain

Based on the CC08-2373 strain constructed in Example 1, MsbA derived from *Salmonella enterica serovar. Typhimurium* (hereinafter referred to as Se.MsbA) was expressed. To this end, the polynucleotide sequence of *S*. *enterica*-derived MsbA obtained based on the sequence (NCBI Reference Sequence: WP_000551246.1) registered in NCBI (National Center for Biotechnology Information Search database) was codon-optimized for *Y. lipolytica* through http://genscript.com. Further, a gene thereof was synthesized (SEQ ID NO: 10) through Macrogen, Inc., and for its expression, a cassette was designed to be inserted at the DPP1 gene locus using TEF promoter (SEQ ID NO: 21) as the promoter, TDH3 terminator (SEQ ID NO: 23) as the terminator, and URA3 gene (SEQ ID NO: 22) as the selection marker.

To this end, the synthesized Se.MsbA gene and the genomic DNA of CC08-2373 were used as template, and PCR was performed using the primers of SEQ ID NO: 46 and SEQ ID NO: 47, SEQ ID NO: 48 and SEQ ID NO: 68, SEQ ID NO: 69 and SEQ ID NO: 70, SEQ ID NO: 71 and SEQ ID NO: 53, SEQ ID NO: 54 and SEQ ID NO: 55, SEQ ID NO: 56 and SEQ ID NO: 57, and SEQ ID NO: 58 and SEQ ID NO: 59 on the left homologous region, TEF promoter, Se.MsbA ORF, TDH3 terminator, URA3, repeat region, and right homologous region fragments, respectively, as shown in Table 6. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR.

**[Table 6]**

| SEQ ID NO | Sequence (5'- 3') | PCR Product |
|---|---|---|
| 46 | AGGTCTTCTGGAACTATTATGTAA | Homology left arm |
| 47 | ATGCGCCGCCAACCCGGTCTCTTCTTTAGCTGTGTTGA AAACAG | |
| 48 | CTGTTTTCAACACAGCTAAAGAAGAGACCGGGTTGGCG GCGCAT | TEF promoter |
| 68 | ACAGGTCCTTATCGTTGTGCATTTTGAATGATTCTTATAC TCAG | |
| 69 | CTGAGTATAAGAATCATTCAAAATGCACAACGATAAGGAC CTGT | Se.MsbA |
| 70 | TTCGCTCTTGATCTTCGGATAGTCACTGGCCAAACTGCA TTTTA | |
| 71 | TAAAATGCAGTTTGGCCAGTGACTATCCGAAGATCAAGA GCGAA | TDH3 terminator |
| 53 | ATGACGAGTCAGACAGGAGGCAGTCTTGGAACGGTGAA AAAGCC | |
| 54 | GGCTTTTCACCGTTCCAAGACTGCCTCCTGTCTGACTC GTCAT | URA3 |
| 55 | TTCGCTCTTGATCTTCGGATAGTGGTGGTATTGTGACTG GGGAT | |
| 56 | ATCCCCAGTCACAATACCACCACTATCCGAAGATCAAGA GCGAA | Repeat region |
| 57 | GATGTATTAACGACAGATTTCAGTCTTGGAACGGTGAAA AAGCC | |
| 58 | GGCTTTTTCACCGTTCCAAGACTGAAATCTGTCGTTAAT ACATC | Homology right arm |
| 59 | CCTGAAAAAAAAACACCTACTCAGC | |
| 32 | GGACATCATCACGTTCAGCTTATCC | Forward |
| 33 | ACCATACAATCGTTTCTGACCAATC | Reverse |

The cassette thus constructed was introduced into the CC08-2373 strain using the heat shock method (D.-C. Chen et al., Appl Microbiol Biotechnol, 1997), and colonies formed on a solid medium lacking uracil (YLMM1; same composition as YLMM1 medium in Example 1) were obtained. The colonies, for which insertion of the cassette into the genome was confirmed using primers of SEQ ID NO: 32 and SEQ ID NO: 33, were cultured on 5-FOA solid medium (same composition as 5-FOA medium in Example 1) at 30°C for 3 days, and by obtaining colonies grown on the 5-FOA solid medium, the URA3 marker was recovered. The final Se.MsbA-expressing strain thus obtained was named CC08-2377.

### Example 7: Deletion of SNQ2 in a retinoid-producing Y. lipolytica strain

Based on the CC08-2373 strain constructed in Example 1, the ORF sequence was removed to inactivate the endogenous SNQ2. To construct an SNQ2 deletion cassette, the genomic DNA of CC08-2373 was used as a template, and PCR was performed using the primers of SEQ ID NO: 72 and SEQ ID NO: 73, SEQ ID NO: 74 and SEQ ID NO: 75, SEQ ID NO: 76 and SEQ ID NO: 77, and SEQ ID NO: 78 and SEQ ID NO: 79 on the left homologous region, URA3, repeat region, and right homologous region fragments, respectively, as shown in Table 7. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR.

**[Table 7]**

| SEQ ID NO | Sequence (5'- 3') | PCR Product |
|---|---|---|
| 72 | ATTCTCATCAGGTAGAGCCACCCACCTTGG | Homology left arm |
| 73 | ATGACGAGTCAGACAGGAGGCAGGTGCTTGGAGTGGT GATATGT | |
| 74 | ACATATCACCACTCCAAGCACCTGCCTCCTGTCTGACTC GTCAT | URA3 |
| 75 | GAAAGCTGTTATGGAAGCTTGTTGGTGGTATTGTGACTG GGGAT | |
| 76 | ATCCCCAGTCACAATACCACCAACAAGCTTCCATAACAG CTTTC | Repeat region |
| 77 | GAATCCTCCGCAAATCCTCCATGGTGCTTGGAGTGGTG ATATGT | |
| 78 | ACATATCACCACTCCAAGCACCATGGAGGATTTGCGGA | Homology right |
| | GGATTC | arm |
| 79 | GACGGGGTTGGGCGACATTGTGGAGTACCG | |
| 80 | GATCGGGTCGGAAAGTGAGATAAA | Forward |
| 81 | TGGGTGTTGAAGACGCCAATGTTC | Reverse |

The SNQ2 deletion cassette thus constructed was introduced into the CC08-2373 strain using the heat shock method (D.-C. Chen et al., Appl Microbiol Biotechnol, 1997), and colonies formed on a solid medium lacking uracil (YLMM1; same composition as YLMM1 medium in Example 1) were obtained. The colonies, for which insertion of the cassette into the genome was confirmed using primers of SEQ ID NO: 80 and SEQ ID NO: 81, were cultured on 5-FOA solid medium (same composition as 5-FOA medium in Example 1) at 30°C for 3 days, and by obtaining colonies grown on the 5-FOA solid medium, the URA3 marker was recovered. The final SNQ2-deleted strain thus obtained was named CC08-2378.

### Example 8: Comparative evaluation of retinoid-excreting ability between the endogenous SNQ2- and exogenous transporter-enhanced strains

To compare the retinoid-producing and excreting abilities of the strains constructed in Examples 1 to 7, a flask evaluation was conducted. Retinoid-producing *Yarrowia lipolytica* strain (CJ2050; KCCM13294P; control group), DPP1-deleted strain constructed in Example 1 (CC08-2373), SNQ2-enhanced strain constructed in Example 2 (CC08-2374), Sc.PDR10-expressing strain constructed in Example 3 (CC08-2488), Sc.SNQ2-expressing strain constructed in Example 4 (CC08-2492), Ec.MsbA-expressing strain constructed in Example 5 (CC08-2376), Se.MsbA-expressing strain constructed in Example 6 (CC08-2377), and SNQ2-deleted strain constructed in Example 7 (CC08-2378) were each inoculated into a 250 mL corner-baffle flask containing 25 mL of YPDLU medium supplemented with 0.05% BHT (3,5-di-tert-4-butylhydroxytoluene) at an initial OD=2, and cultured at 30°C and 200 rpm for 48 hours. The YPDLU medium used had the following composition:
<YPDLU>
Glucose 40 g/L, Bacto peptone 20g/L, Yeast extract 10g/L, Uracil 1g/L, Leucine 1g/L, 1M Phosphate buffer (pH 7.0) 100ml/L

To evaluate the growth level of each strain, OD values were measured at a wavelength of 600 nm using a spectrophotometer. The concentrations of retinol, retinal, and beta-carotene excreted extracellularly by each strain were quantified by mixing 0.1 mL of the supernatant obtained by removing cells from the culture broth after completion of culturing with 0.9 mL of acetone containing 4% BHT (Sigma), followed by analysis using HPLC equipment.

The analyzed OD values and concentrations of retinoid and beta-carotene are as shown in Table 8. The retinoid concentrations are schematically illustrated in FIG. 1.

**[Table 8]**

| Strain | OD(A₆₀₀) | Extracellular | | | -fold | |
|---|---|---|---|---|---|---|
| | | Retinol (µg/L) | Retinal (µg/L) | β-carotene (µg/L) | Retinol | Retinal |
| CC08-2050 | 98 | 9790 | 2390 | N.D | 1.0 | 1.0 |
| CC08-2373 | 97 | 10800 | 2300 | N.D | 1.1 | 1.0 |
| CC08-2374 | 99 | 26190 | 3400 | N.D | 2.7 | 1.4 |
| CC08-2488 | 103 | 7540 | 1330 | N.D | 0.8 | 0.6 |
| CC08-2492 | 110 | 6090 | 1530 | N.D | 0.6 | 0.6 |
| CC08-2376 | 90 | 9880 | 2300 | N.D | 1.0 | 1.0 |
| CC08-2377 | 103 | 9130 | 2140 | N.D | 0.9 | 0.9 |
| CC08-2378 | 101 | 1317 | 198 | N.D | 0.1 | 0.1 |

Comparing the values of CC08-2050 (control strain) and CC08-2373 (DPP1-deleted strain) in Table 8, it was confirmed that the deletion of DPP1 for foreign gene insertion does not affect retinoid production. Accordingly, evaluations were conducted on the *Yarrowia lipolytica* strain with enhanced endogenous SNQ2 (CC08-2374) and *Yarrowia lipolytica* strains introduced with ABC transporters derived from other microorganisms (*S*. *cerevisiae, E. coli,* and *S*. *enterica*) (CC08-2488, CC08-2492, CC08-2376, CC08-2377) based on Strain CC08-2373.

As a result, the amounts of extracellularly excreted retinol and retinal increased compared to the control by 2.7-fold and 1.4-fold, respectively, only when the endogenous SNQ2 of *Yarrowia lipolytica* was enhanced (CC08-2374; SNQ2-enhanced strain). In contrast, when SNQ2 was deleted (CC08-2378), the amounts of retinol and retinal excreted from the microorganism each decreased to about 0.1-fold compared to the control.

The above results suggest that the enhancement of endogenous SNQ2 plays a key role in the excretion of retinoid in *Yarrowia lipolytica.* It is further suggested that since beta-carotene was not detected in the culture broth, the SNQ2 protein of *Yarrowia lipolytica* selectively excretes retinoids.

From the foregoing description, a person skilled in the art to which the present disclosure pertains will be able to understand that the present disclosure may be embodied in other specific forms without modifying the technical concepts or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts fall within the scope of the present disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-2050 | Accession number given by the
INTERNATIONAL DEPOSITARY AUTHORITY:
KCCM13294P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 01. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 01. 2022.

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950. 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea Tel: 82-2-391-0950, 396-0950 Fax: 82-2-392-2859

## Claims

1. A *Yarrowia lipolytica* microorganism having retinoid-producing ability with enhanced activity of an SNQ2 protein.

2. The microorganism according to claim 1, wherein the SNQ2 protein comprises SEQ ID NO: 1 or an amino acid sequence having 90% or more homology thereto.

3. The microorganism according to claim 1, wherein the SNQ2 protein is derived from *Yarrowia lipolytica.*

4. The microorganism according to claim 1, wherein the protein is encoded by an SNQ2 gene of SEQ ID NO: 2 or a polynucleotide sequence having 90% or more homology thereto.

5. The microorganism according to claim 1, wherein the microorganism has enhanced retinoid-excreting ability compared to a *Yarrowia lipolytica* microorganism having retinoid-producing ability without enhanced activity of an SNQ2 protein.

6. The microorganism according to claim 1, wherein the microorganism further comprises lycopene cyclase/phytoene synthase comprising SEQ ID NO: 11 or an amino acid sequence having 90% or more homology thereto.

7. The microorganism according to claim 1, wherein the microorganism further comprises phytoene desaturase comprising SEQ ID NO: 13 or an amino acid sequence having 90% or more homology thereto.

8. The microorganism according to claim 1, wherein the microorganism further comprises geranylgeranyl pyrophosphate synthase comprising SEQ ID NO: 15 or an amino acid sequence having 90% or more homology thereto.

9. The microorganism according to claim 8, wherein the geranylgeranyl pyrophosphate synthase is derived from *Haematococcus pluvialis.*

10. The microorganism according to claim 1, wherein the microorganism further comprises beta-carotene 15,15'-oxygenase comprising SEQ ID NO: 17 or an amino acid sequence having 90% or more homology thereto.

11. The microorganism according to claim 1, wherein the retinoid comprises any one selected from the group consisting of retinol, retinal, retinoic acid, and retinyl ester.

12. A method for producing retinoid, comprising culturing the microorganism of any one of claims 1 to 11 in a medium.

13. The method according to claim 12, comprising recovering retinoid from the cultured medium or microorganism.

14. The method according to claim 12, wherein the method, during retinoid extraction, does not utilize microbial cell disruption or use dodecane as a solvent.

15. The method according to claim 12, wherein the retinoid comprises any one selected from the group consisting of retinol, retinal, retinoic acid, and retinyl ester.

16. A method for preparing a *Yarrowia lipolytica* microorganism having retinoid-producing ability, comprising enhancing the activity of an SNQ2 protein in the *Yarrowia lipolytica* microorganism having retinoid-producing ability.

17. A method for increasing retinoid excretion, comprising enhancing the activity of an SNQ2 protein in a *Yarrowia lipolytica* microorganism having retinoid-producing ability.

18. A composition for producing retinoid, comprising one or more of a *Yarrowia lipolytica* microorganism having retinoid-producing ability with enhanced activity of an SNQ2 protein, and a culture thereof.

19. Use of a *Yarrowia lipolytica* microorganism with enhanced activity of an SNQ2 protein for retinoid production.
